(19) Europäisches Patentamt
European Patent Office
Office européen des brevets

(11) Veröffentlichungsnummer: **0 023 885**
**B1**

(12) # EUROPÄISCHE PATENTSCHRIFT

(45) Veröffentlichungstag der Patentschrift: **12.12.84**

(51) Int. Cl.³: **C 07 D 498/08**, A 61 K 31/71

(21) Anmeldenummer: **80810227.1**

(22) Anmeldetag: **14.07.80**

(54) **Verfahren zur Einführung einer sauerstoffhaltigen funktionellen Gruppe in Ansamycinen, danach erhaltene Verbindungen, sowie sie enthaltende pharmazeutische Präparate.**

(30) Priorität: **20.07.79 CH 6774/79**

(43) Veröffentlichungstag der Anmeldung: **11.02.81 Patentblatt 81/6**

(45) Bekanntmachung des Hinweises auf die Patenterteilung: **12.12.84 Patentblatt 84/50**

(84) Benannte Vertragsstaaten: **CH DE FR GB IT LI**

(56) Entgegenhaltungen:
**EP-A-0 014 181**
**CH-A-605 974**
**DE-A-2 548 128**
**FR-A-1 490 183**

**Rodd's Chemistry of Carbon Compounds (1974), Band III Teil B, S. 90**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Postfach, CH-4002 Basel (CH)**

(72) Erfinder: **Kump, Wilhelm, Dr., Friedrich-Oser-Strasse 10, CH-4105 Biel-Benken (CH)**

**EP 0 023 885 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft in erster Linie die Einführung einer sauerstoffhaltigen funktionellen Gruppe in das Molekül von Ansamycinen, und zwar einer freien, verätherten oder veresterten Hydroxylgruppe in die 3-Stellung der Ansamycine vom Rifamycin-Typ.

Man kennt bereits Rifamycin-S-Derivate mit Substituenten in 3-Stellung, nämlich die entsprechenden 3-Aminoderivate gemäß CH-A-605 974 und FR-A-1 490 183, sowie entsprechende 3-Halogenderivate gemäß DE-A-2 548 128. Wie kürzlich gefunden wurde, vgl. unsere EP-A-14 181, weist das neue, auf fermentativem Wege gewonnene 3-Hydroxyrifamycin-S günstige antibiotische, insbesondere antimikrobielle Eigenschaften auf, die denjenigen von Rifamycin-S im Charakter ähnlich sind.

In der Suche nach einem alternativen, rein chemischen Zugang zu dieser Verbindung wurde nun das vorliegende erfindungsgemäße Verfahren gefunden, welches auf eine überraschend einfache Weise nicht nur das gewünschte vorbekannte 3-Hydoxyrifamycin-S zu liefern vermag, sondern auch den Zugang zu einer breiten Gruppe von neuen Rifamycin-Analogen erschließt, welche sich durch ein bisher nicht bekanntes Strukturmerkmal, und zwar eine freie, verätherte oder veresterte Hydroxylgruppe in 3-Stellung, auszeichnen.

Die verfahrensgemäß erhältlichen Endstoffe sind Verbindungen der Formel (I)

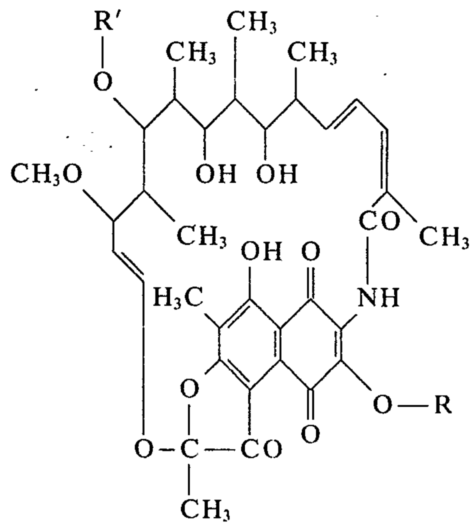

(I)

worin R und R' je ein Wasserstoffatom oder R' Acetyl und R Wasserstoff oder ein Rest Alk, Ar, Alk—CO—, Ar—CO— oder H—CO— ist, wobei Alk einen Alkylrest mit 1—7 C-Atomen, 2-Methoxyäthyl, 2-Äthoxyäthyl, 2-Hydroxyäthyl, 2- oder 3-Hydroxypropyl oder 4-Hydroxybutyl, in welchen die Hydroxylgruppe auch durch Ameisensäure oder eine $C_1$—$C_7$-Alkan-carbonsäure verestert werden kann, und Ar einen gegebenenfalls durch $C_1$—$C_7$-Alkyl, $C_1$—$C_7$-Alkoxy, Methylendioxy oder Formyl substituierten Phenylrest darstellt, sowie die entsprechenden Rifamycin-SV-Derivate mit der 1,4-Hydrochinon-Struktur.

Infolge der sehr engen Beziehung zwischen der 1,4-Chinon- und -Hydrochinon-Form (entsprechend Rifamycin-S und SV) und der Leichtigkeit, mit welcher die beiden Formen ineinander übergehen, sind überall, wo nicht spezifisch anders angegeben, beide Formen inbegriffen.

Wenn nicht anders angegeben, enthalten die organischen Reste höchstens 7, vorzugsweise höchstens 4 Kohlenstoffatome.

Das erfindungsgemäße Verfahren ist dadurch gekennzeichnet, daß man in 3-Bromrifamycin-S der Formel (II)

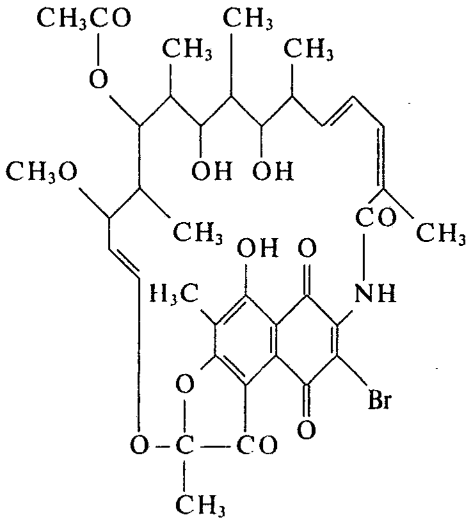

(II)

Brom gegen die oben definierte Gruppe OR austauscht und das Produkt in Form eines Derivats der S-Reihe oder SV-Reihe isoliert. Der Austausch erfolgt dadurch, daß man die Verbindung der Formel (II) mit einer Verbindung der Formel ROH (III), worin R die obengenannten Bedeutungen hat, oder einem Salz davon in neutralem bis basischem Milieu und Temperaturen zwischen 20° bis 140° und gegebenenfalls in Anwesenheit eines Bromwasserstoff-bindenden Mittels und/oder eines organischen Lösungsmittels behandelt.

Die praktische Durchführung des erfindungsgemäßen Verfahrens erfolgt unter den konventionellen allgemeinen Bedingungen der präparativen organischen Chemie: im Normalfall arbeitet man unter Atmosphärendruck oder einem leicht erhöhten Druck, z. B. bei leichtflüchtigen Lösungsmitteln zum Erreichen von höheren Reaktionstemperaturen; diese liegen zwischen 20 bis 140°C, vorzugsweise zwischen 90 bis 125°C. Üblicherweise nimmt man einen Überschuß, z. B. 2 bis 15 Moläquivalente, des Austauschmittels der Formel (III) und gegebenenfalls auch an Hilfsmitteln, und führt die Reaktion in homogener oder heterogener Phase, d. h. in Lösung oder Suspension, im letzteren Fall vorzugsweise unter Rühren, durch. Normalerweise erstreckt sich die Reaktionszeit über einen Zeitraum von 30 Minuten bis einem Tag, was von der Reaktionstemperatur und den übrigen Bedingungen sowie von den spezifischen Eigenschaften der Komponente (III) abhängig ist. Der Fortschritt der Reaktion kann durch die konventionellen analytischen Methoden, insbesondere durch Spektroskopie und Dünnschichtchromatographie, kontrolliert werden.

Das Symbol R im Austauschreagens der Formel ROH (III) kann jede der obengenannten allgemeinen und bevorzugten Bedeutungen haben. Der Ausgangsstoff der Formel (II), d. h. 3-Bromrifamycin-S, ist bekannt, vgl. DE-A-2 548 128 (Marsili et al.).

Als Hilfsmittel zum Binden von Bromwasserstoff können im Prinzip beliebige basische Verbindungen zugezogen werden, wie einerseits organische stickstoffhaltige Basen, z. B. tertiäre Amine vom Typ Triäthylamin, Äthyldiisopropylamin, N-Äthylpiperidin oder N,N'-Dimethylpiperazin, oder aromatische heterocyclische Basen vom Typ Pyridin, Collidin oder Chinolin, andererseits basisch reagierende anorganische Verbindungen, insbesondere Alkalimetallsalze schwacher oder mittelstarker Säuren, z. B. die weiter unten genannten, sowie analoge Salze von Carbonsäuren; ferner auch Ionenaustauscher, wie insbesondere Kationenaustauscher im Alkalimetall-, wie Na- oder K-, -Cyclus oder Anionenaustauscher im OH-Cyclus. Schließlich können, mindestens teilweise, diesem Zwecke neutral reagierende stickstoffhaltige Verbindungen dienen, die zugleich oft auch vorteilhafte Lösungsmittel darstellen, z. B. Carbonsäureamide, insbesondere niederaliphatische Carbonsäureamide, wie Formamid, N,N-Dimethylformamid, Acetamid und N,N-Dimethylacetamid, und cyclische Amide, wie N-Methylpyrrolidon, sowie Amidoderivate der Kohlensäure, wie Urethane. Bei der Wahl des spezifischen Mittels ist notwendig, die große Empfindlichkeit der Rifamycin-Struktur immer zu berücksichtigen und solche Mittel zu vermeiden, welche zu Nebenreaktionen führen würden. — Die obengenannten basischen Verbindungen können zur Einhaltung der basischen Reaktion des Milieus dienen; speziell für diesen Zweck kann man die üblichen Puffer verwenden.

Dabei ist auch zu bemerken, daß eine und dieselbe Reaktionskomponente auch die Funktion einer anderen oder mehrerer Komponenten bzw. Hilfsstoffe übernehmen kann, z. B. das Reaktionsmittel der Formel (III) zugleich als Lösungsmittel verwendet werden, oder eine basische Komponente nicht nur zur Einstellung der basischen Reaktion dienen, sondern durch den Puffereffekt auch zugleich Bromwasserstoff binden, oder sogar, wie z. B. ein Metallsalz einer Carbonsäure, neben diesen zwei Funktionen auch noch das eigentliche Austauschmittel (III) darstellen.

Obwohl der Austauschreaktion immer dasselbe Prinzip zugrundeliegt und die Umsetzung nach einem einheitlichen Grundschema erfolgt, ist es für ein optimales Resultat notwendig, bei praktischer Durchführung die Eigenart der Reaktionskomponenten, in erster Linie die des jeweiligen Reaktionsmittels der

Formel (III), zu berücksichtigen. Gemäß der vorangehenden Definition des Restes R kann die Formel ROH (III) Wasser, einen Alkohol und eine Säure, bzw. ein Salz davon, darstellen, also Verbindungen, deren physikalische Eigenschaften, z. B. Löslichkeit und Mischbarkeit, sowie das allgemeine chemische Verhalten, oft weit auseinander liegen und somit den Verlauf der erfindungsgemäßen Umsetzung wesentlich beeinflussen können. Diesem Umstand zufolge sind die für jeden solchen Verbindungstyp besonders vorteilhaften, bevorzugten Verfahrensbedingungen spezifisch zu wählen.

Soll durch den erfindungsgemäßen Austausch freies Hydroxyl (R=H) eingeführt werden, wird die Reaktion in wäßrigem Milieu durchgeführt; zur Verbesserung der Löslichkeitsverhältnisse kann man dem Reaktionsgemisch organische Lösungsmittel beimischen, z.B. Alkohole, insbesondere Niederalkanole wie Methanol, Äthanol oder Isopropylalkohol, oder andere mit Wasser mischbare hochpolare aprotische Lösungsmittel vom Typ Dimethylsulfoxid, Dimethylformamid oder Hexamethylphosphortriamid. Die Umsetzung erfolgt unter schwach basischen Bedingungen, insbesondere beim pH zwischen 7,0 bis 10,0, wobei man unter Umständen durch eine engere Auswahl des pH-Wertes den Verlauf der Umsetzung noch weiter lenken kann. Arbeitet man im pH-Bereich um 9,0, so erhält man vorwiegend das 25-O-Desacetyl-3-hydroxyrifamycin-S (I; R=R'=H), beim pH unterhalb 8,0 wird dagegen die 25ständige Estergruppierung nicht angegriffen, und es resultiert das 3-Hydroxyrifamycin-S. Zum Einstellen des gewünschten pH-Wertes verwendet man dabei mit Vorteil Salze von Alkalimetallen, wie insbesondere von Natrium und Kalium, mit schwachen oder mittelstarken anorganischen und organischen Säuren, insbesondere solchen, die man zur Zubereitung von Puffer-Lösungen verwendet, wie z. B. Hydrocarbonate, Carbonate, primäre, sekundäre und tertiäre Phosphate, Acetate, Benzoate, Zitrate und Tartrate. Diese Salze dienen vorzugsweise zugleich auch als Bromwasserstoff-bindende Mittel, vorausgesetzt, daß sie in einer Menge eingesetzt werden, die nicht nur die Acidität des Bromwasserstoffs zu neutralisieren (wozu an sich ein Moläquivalent genügen würde), sondern weiterhin das pH im gewünschten Bereich zu halten vermag; vorzugsweise unterschreitet diese Menge nicht 2 Moläquivalente. Wünscht man die gleichzeitige Desacetylierung in 25-Stellung, verwendet man vorzugsweise Natriumhydrocarbonat, Natriumcarbonat oder Kaliumcarbonat, soll dagegen die 25-O-Acetylgruppe erhalten bleiben, greift man vornehmlich zu Natriumacetat oder einem geeigneten Phosphat-Puffer.

Soll durch den erfindungsgemäßen Austausch eine 3-Äthergruppe OR (R = Alk oder Ar) entstehen, so arbeitet man vorwiegend in überschüssigem Reaktionsmittel, d. h. im entsprechenden Alkohol AlkOH bzw. ArOH, der zugleich als Lösungsmittel funktioniert, und in Abwesenheit von Wasser. Als das Bromwasserstoff-bindende Mittel kann man eines der obengenannten, vor allem jedoch ein Alkalimetallsalz einer Carbonsäure, wie insbesondere Natriumacetat, verwenden, welches dann dem Gemisch auch die notwendige schwach basische Reaktion erteilt. Vornehmlich ist jedoch nur eine minimale Menge, nicht überschreitend etwa 2 Moläquivalente, dieses Hilfsmittels einzusetzen, um Nebenreaktionen, hauptsächlich den Austausch von Brom gegen die Acetoxygruppe, in Grenzen zu halten.

Soll durch den erfindungsgemäßen Austausch eine veresterte 3-Hydroxylgruppe OCOH, OCOAlk oder OCOAr eingeführt werden, so nimmt man als das Austauschmittel vorzugsweise ein Alkalimetallsalz der einzuführenden Carbonsäure, vor allem das Natriumsalz, welches man vornehmlich in einem Überschuß, d. h. in einer Menge von mindestens 2 Moläquivalenten, einsetzt. (Wie oben gesagt, bindet dabei das Reagens zugleich den entwickelten Bromwasserstoff und erteilt dem Gemisch die schwach basische Reaktion). Man kann aber auch eine freie Säure einsetzen und ihr Salz mittels einer äquivalenten Menge eines Alkalimetall-hydrocarbonats oder -carbonats direkt im Reaktionsgemisch vorübergehend entstehen lassen. Als Lösungsmittel verwendet man vorzugsweise ein wasserfreies aprotisches polares Lösungsmittel, wie insbesondere ein Niederalkansäureamid, z. B. N,N-Dimethylacetamid, N,N-Diäthylacetamid und vor allem N,N-Dimethylformamid, ein Diniederalkylsulfoxid, wie Dimethylsulfoxid, oder Hexamethylphosphortriamid.

Die Ausgangsstoffe der Formel ROH sind bekannte Verbindungen oder analog zu diesen durch bekannte Verfahren zugänglich.

Im primären Reaktionsgemisch liegen üblicherweise beide Oxidationsstufen des Endstoffes, d. h. die 1,4-Chinon-Form der S-Reihe und die 1,4-Hydrochinon-Form der SV-Reihe, nebeneinander vor. Zweckmäßig isoliert man jedoch das gesamte Produkt in nur einer einzigen der beiden Formen, vorzugsweise in der Chinon-Form. Zu diesem Zwecke behandelt man das rohe Reaktionsgemisch mit einem Oxidationsmittel, insbesondere einem solchen, das für die Oxidation bekannter Hydrochinone üblich ist, z.B. Ammoniumpersulfat, Wasserstoffperoxid, Luftsauerstoff oder vorzugsweise Kaliumferricyanid; die Oxidation erfolgt vornehmlich unter basischen Bedingungen. Wünscht man das Endprodukt in der Hydrochinon-Form (als ein Rifamycin-SV-derivat) zu isolieren, behandelt man das rohe Reaktionsgemisch mit einem üblichen Chinon-Reduktionsmittel, wie Hydrosulfit, Dithionit oder insbesondere Ascorbinsäure oder Zink-Eisessig. In derselben Weise kann man auch individuelle Endstoffe beider Reihen ineinander überführen.

Die vorliegende Erfindung umfaßt auch das vorbekannte 3-Hydroxyrifamycin, sofern es durch das erfindungsgemäße Verfahren hergestellt wird, und insbesondere die neuen 3-Hydroxyrifamycin-S-Derivate der allgemeinen Formel (I)

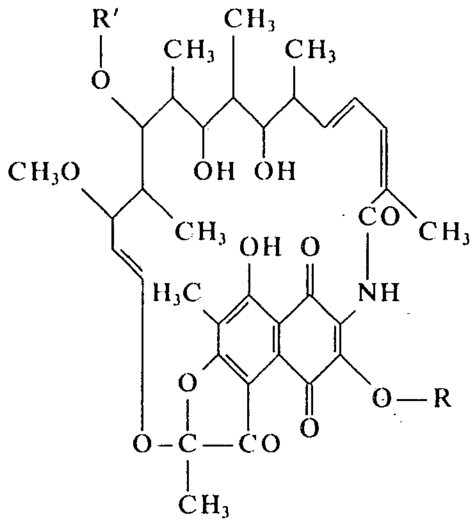

(I)

worin R und R' je ein Wasserstoffatom darstellt oder R' für Acetyl und R ein Rest Alk, Ar, Alk—CO—, Ar—CO— oder H—CO— ist, wobei Alk einen Alkylrest mit 1—7 C-Atomen, 2-Methoxyäthyl, 2-Äthoxyäthyl, 2-Hydroxyäthyl, 2- oder 3-Hydroxypropyl oder 4-Hydroxybutyl, in welchen die Hydroxylgruppe auch durch Ameisensäure oder eine $C_1$—$C_7$-Alkan-carbonsäure verestert werden kann, und Ar einen gegebenenfalls durch $C_1$—$C_7$-Alkyl, $C_1$—$C_7$-Alkoxy, Methylendioxy oder Formyl substituierten Phenylrest darstellt, und die entsprechenden Rifamycin-SV-Derivate mit der 1,4-Hydrochinon-Struktur, sowie pharmazeutische Zusammensetzungen und Präparate in Dosierform, die diese 3-Hydroxyrifamycin-S-Derivate enthalten, und Verfahren zur Herstellung derselben. Gegenstand der Erfindung sind auch die neuen 3-Hydroxyrifamycin-S-Derivate der Formel (I) zur Verwendung als Antibiotika, insbesondere antimikrobielle, vor allem gegen Kokken und Bakterien wirkende Mittel.

Die oben charakterisierten erfindungsgemäßen 3-Hydroxyrifamycin-S-Derivate können dank ihren antibiotischen, insbesondere antimikrobiellen Eigenschaften als wertvolle Chemotherapeutika in diesem Bereich Verwendung finden. Sie können auch als Zusatz für Futtermittel und zur Konservierung von Nahrungsmitteln sowie als Desinfektionsmittel verwendet werden. Ferner können sie auch als Zwischenprodukte für die Herstellung anderer nützlicher Verbindungen, insbesondere von antibiotisch wirksamen Rifamycin-Derivaten dienen.

Ist Alk eine veresterte 2-Hydroxyäthyl-, 2- und 3-Hydroxypropyl- oder 4-Hydroxybutylgruppe, so handelt es sich insbesondere um die acetylierte Form; speziell genannt seien 2-Formyloxyäthyl und 2-Acetoxyäthyl.

Eine in veresterter Form vorliegende Hydroxylgruppe ist eine solche, worin das Wasserstoffatom der Hydroxylgruppe durch den Acylrest $-COR^1$ ersetzt ist. In dieser Formel kann $R^1$ entweder für Wasserstoff stehen und somit den Formylrest bilden, oder $R^1CO$— ist der Rest einer $C_{1-7}$-Alkancarbonsäure, wir der Propion-, Butter-, Isobutter-, Valerian-, Isovalerian-, Capron-, Trimethylessig-, Oenanth- und Diäthylessigsäure und vor allem der Essigsäure.

Im Acyl Alk—CO— oder Ar—CO— kann Alk bzw. Ar alle angegebenen Bedeutungen Alk und Ar haben, vorzugsweise ist Alk—CO— Acetyl, Propionyl und Butyryl. Unter bevorzugten Resten Ar—CO— sind insbesondere zu nennen: Benzoyl, p-Methoxybenzoyl, o-, m- und p-Toluoyl und 2,4,6-Trimethylbenzoyl.

Diese neuen Verbindungen zeichnen sich durch ihre wertvollen pharmakologischen Eigenschaften aus: so besitzen sie gemäß den Resultaten in vitro antibiotische, insbesondere antibakterielle Eigenschaften, wie z.B. einerseits gegen grammpositive und grammnegative Kokken, wie Staphylococcus aureus, Streptococcus pyogenes, Streptococcus pneumoniae, Neisseria meningitis, Neisseria gonorhoae (im getesteten Konzentrationsbereich 0,01 bis 8 mcg/ml), andererseits gegen grammnegative Stäbchenbakterien, wie Enterobacteriaceae, Pseudomonas aeruginosa, Haemophilus influenzae (im getesteten Konzentrationsbereich von 0,01 bis 64 mg/ml). Besonders hervorzuheben als Wirkstoffe sind 3-Acetoxy-, 3-Benzoyl-, 3-Phenoxy-, 3-(2-Methoxyäthyl)- und vor allem 3-Methoxyrifamycin-S, die alle deutliche Hemmwirkung schon bei der genannten untersten Konzentration aufweisen.

In Anlehnung an diese günstigen Eigenschaften verwendet man die erfindungsgemäßen neuen 3-Hydroxyrifamycin-S-Derivate, allein oder in Kombination mit einem anderen Antibiotikum oder Chemotherapeutikum, als ein Mittel zur Bekämpfung von Infektionen, die durch Bakterien oder Kokken, z.B. die genannten, hervorgerufen werden, und zwar sowohl als Heilmittel, wie auch als Desinfektionsmittel. Bei der Verwendung als Heilmittel wird der erfindungsgemäße Wirkstoff vorzugsweise in Form eines pharmazeutischen Präparats zusammen mit mindestens einem konventionellen pharmazeutischen Träger oder Hilfsstoff einem Warmblüter, vor allem Menschen, verabreicht.

Zwecks Herstellung pharmazeutischer Präparate kann jede einzelne der erfindungsgemäßen Verbindungen, vor allem eine der besonders hervorgehobenen, mit einem für die topische, enterale oder parenterale Applikation geeigneten anorganischen oder organischen Trägermaterial vermischt werden.

Für dasselbe kommen solche Stoffe in Betracht, die mit der neuen Verbindung nicht reagieren, wie z. B. Gelatine, Milchzucker, Stärke, Magnesiumstearat, pflanzliche Öle, Benzylalkohol, oder andere Arzneimittelträger. Die pharmazeutischen Präparate können z. B. als Tabletten, Dragées, Pulver, Suppositorien, oder in flüssiger Form als Lösungen, Suspensionen, Emulsionen, Cremen oder Salben vorliegen. Gegebenenfalls sind sie sterilisiert und/oder enthalten Hilfsstoffe, wie Konservierungsmittel, Stabilisierungs-, Netz- oder Emulgiermittel. Sie können auch noch andere therapeutisch wertvolle Stoffe enthalten. Auch die Desinfektionsmittel können mit geeigneten Trägerstoffen, wie bekannt, vermischt werden.

Die Dosierung der Wirkstoffe, z. B. der oben besonders hervorgehobenen 3-Hydroxyrifamycin-S-Derivate, erfolgt im Prinzip analog derjenigen von anerkannten Antibiotika vom Rifamycin-Typ; sie hängt jedoch auch einerseits von Spezies, Körpergewicht, Alter und individuellem Zustand des Warmblüters, andererseits von der Applikationsweise und insbesondere von der jeweiligen Empfindlichkeit des Krankheitserregers ab, die man im Routine-Test in bekannter Weise feststellen kann.

Die Erfindung betrifft auch eine Methode zur Tötung oder Wachstumsbehinderung (d. h. Inhibition) eines gegen mindestens ein der erfindungsgemäßen 3-Hydroxyrifamycin-S-Derivate empfindlichen Mikroorganismus, welche durch die Behandlung dieses Mikroorganismus oder eines durch diesen Mikroorganismus infizierten Medium mit einer antimikrobiell wirksamen Dosis einer der erfindungsgemäßen 3-Hydroxyrifamycin-S-Derivate charakterisiert ist. – Unter der Bezeichnung »eine antimikrobiell wirksame Dosis« ist eine solche Menge des Wirkstoffes zu verstehen, die zu einer wirkungsvollen Inhibition des betreffenden zu behandelnden Mikroorganismus ausreicht.

Die nachfolgenden Beispiele illustrieren die obenbeschriebene Erfindung. Temperaturen werden in Celsiusgraden angegeben. Die Zusammensetzung von Lösungsmittelgemischen ist im Volumenverhältnis angegeben.

### Beispiel 1

Eine Lösung von 3,0 g 3-Bromrifamycin-S in 50 ml Methanol und 70 ml Phosphatpuffer vom pH 8,0 (0,065 Mol $Na_2HPO_4$ und 0,004 Mol $KH_2PO_4$ pro Liter Wasser) wird 4 Stunden unter Rückfluß erhitzt. Dabei verändert sich die Farbe der Reaktionslösung von blauviolett nach gelbbraun. Die Reaktionslösung wird darauf mit überschüssigem Kaliumferricyanid oxydiert und nach dem Ansäuern auf pH ~ 3 mit Chloroform extrahiert. Nach dem Trocknen und Eindampfen des Chloroformextrakts wird der Rückstand in Äther gelöst und die Ätherlösung erschöpfend mit Phosphatpuffer vom pH = 6 extrahiert. Der weinrote wäßrige Pufferextrakt wird bis zum Farbumschlag nach gelb angesäuert und das ausgeschiedene 3-Hydroxyrifamycin-S mit Chloroform aufgenommen. Nach dem Abdampfen des Chloroforms resultiert in Form eines gelben Schaums 3-Hydroxyrifamycin-S, dessen Identität mit dem früher erhaltenen Fermentationsprodukt sich anhand von Dünnschichtchromatographie, 360-MHz-$^1$H-NMR-Spektren sowie von UV- und IR-Spektren sicherstellen läßt.

### Beispiel 2

Ein Gemisch aus 1,0 g 3-Bromrifamycin-S, 0,5 g wasserfreiem Natriumacetat und 30 ml Methanol wird im Druckrohr 5 Stunden auf 75° erhitzt. Das Reaktionsgemisch wird eingedampft, mit Zitronensäure angesäuert und an Kieselgel mit Chloroform-Aceton (19 : 1) als Elutionsmittel chromatographiert. Das zuerst eluierte Material besteht aus reinem 3-Methoxyrifamycin-S; 100-MHz-$^1$H-NMR-Spektrum (in $CDCl_3$): kein Signal für H-3; 4,16 ppm (s, 3 H, $OCH_3$ an C-3); UV-Spektrum (EtOH; nm/$\varepsilon_{max}$): 215/26900, 268/23400, ~ 300 (Schulter), ~ 350 (Schulter), 396/4360; IR-Spektrum ($CH_2Cl_2$): 3500 (OH), 3400 (NHCO), 1735 (Ester, 5-Ringketon), 1670 (Amid-I, Chinon-CO), 1640 (Chinon-CO verbrückt) cm$^{-1}$; Massenspektrum: m/e = 725 ($C_{38}H_{47}NO_{13}$), starke Fragmente: 709, 693, 695, 635, 303, 423.

### Beispiel 3

Eine Lösung von 1,0 g 3-Bromrifamycin-S in 30 ml 2-Methoxyäthanol, welche 0,5 g wasserfreies Natriumacetat suspendiert enthält, wird eine Stunde auf 90° unter Rühren erhitzt und eingedampft. Der Rückstand wird mit Zitronensäure behandelt und an Kieselgel mit Methylenchlorid-Aceton (19 : 1) als Elutionsmittel chromatographiert. Das erhaltene 3-(2-Methoxyäthoxy)-rifamycin-S ist folgendermaßen charakterisiert: 100-MHz-$^1$H-NMR-Spektrum (in $CDCl_3$): Signale des C-3 Substituenten bei 3,36 (s, 3 H, $OCH_3$), ~ 3,7 (m) und 4,62 (m, $OCH_2CH_2OCH_3$) ppm.

### Beispiel 4

Eine Lösung von 1,5 g 3-Bromrifamycin-S und 0,75 g Phenol in 30 ml Dimethylsulfoxid wird mit 0,75 g wasserfreiem Natriumacetat versetzt und während 3 Stunden auf 90°C erhitzt. Nach Erkalten

wird das Reaktionsgemisch mit wäßriger Kaliumferricyanidlösung im Überschuß versetzt und mit Zitronensäure angesäuert; die gebildeten Reaktionsprodukte werden in Chloroform aufgenommen. Das im Chloroformextrakt enthaltene Material liefert durch die Chromatographie an Kieselgel mit Chloroform-Aceton (19 : 1) zunächst 3-Bromrifamycin-S, danach das gewünschte 3-Phenoxyrifamycin-S; 100-MHz-$^1$H-NMR-Spektrum (in $CDCl_3$): Signale des Phenoxysubstituenten bei 6,8—7,4 (m, 5 H) ppm; UV-Spektrum (0,01-N alkoholische Salzsäure, nm/$\varepsilon_{max}$): 220/34600, 266 (Schulter), 270/24800, 342/6000, 400 (Schulter).

## Beispiel 5

Ein Gemisch von 2,0 g 3-Bromrifamycin-S mit 2 g wasserfreiem Natriumacetat und 50 ml Dimethylsulfoxid wird eine Stunde auf 90° erhitzt. Dabei ändert sich die anfangs blauviolette Farbe des Reaktionsgemisches nach braunrot. Anschließend setzt man wäßrige Kaliumferricyanidlösung im Überschuß zu, säuert mit Zitronensäure an und nimmt die gebildeten Reaktionsprodukte mit Äther auf. Aus der Ätherlösung wird 3-Acetoxyrifamycin-S durch wiederholtes Ausschütteln mit Phosphatpuffer vom pH 6,0 extrahiert. Die vereinigten wäßrigen Pufferextrakte werden mit Zitronensäure angesäuert und das ausgeschiedene 3-Acetoxyrifamycin-S mit Chloroform aufgenommen. Nach dem Trocknen und Eindampfen des Chloroformextrakts erhält man praktisch reines 3-Acetoxyrifamycin-S, das aus Äther in hellgelben Nadeln kristallisiert, Smp. 154—155°. Massenspektrum: m/e = 753 ($C_{39}H_{47}NO_{14}$), charakteristische Fragmente bei: 723, 721, 713, 711, 705, 695, 693, 663, 661. 100-MHz-$^1$H-NMR-Spektrum (in $CDCl_3$): Signale von 2-Acetylgruppen bei 2,10 (s, 6 H) ppm; UV-Spektrum (0,01-N alkoholische Salzsäure, nm/$\varepsilon_{max}$): 226/33100, 273/26700, 340/6200, 390/4260.

## Beispiel 6

Ein Gemisch von 2,0 g 3-Bromrifamycin-S mit 2 g wasserfreiem Natriumbenzoat und 30 ml Dimethylformamid wird während 2 ½ Stunden auf 70° erhitzt. Dabei ändert das anfangs blauviolett gefärbte Reaktionsgemisch seine Farbe nach braunrot. Anschließend setzt man wäßrige Kaliumferricyanidlösung im Überschuß zu, säuert mit Zitronensäure an und nimmt die gebildeten Reaktionsprodukte mit Äther auf. Aus der Ätherlösung wird das 3-Benzoyloxyrifamycin-S durch wiederholtes Ausschütteln mit Phosphatpuffer vom pH = 6,0 extrahiert. Die vereinigten wäßrigen Pufferextrakte werden mit Zitronensäure angesäuert und das ausgeschiedene 3-Benzoyloxyrifamycin-S mit Chloroform aufgenommen. Nach dem Trocknen und Eindampfen des Chloroformextrakts erhält man 3-Benzoyloxyrifamycin-S; Massenspektrum: nach Silylierung m/c 1031 (Tris-trimethylsilylderivat von 3-Benzoyloxyrifamycin-S). 100-MHz-$^1$H-NMR-Spektrum (in $CDCl_3$): Signale der Benzoylgruppe bei 7,3—8,1 (m, 5 H) ppm.

## Beispiel 7

Ein Gemisch von 2,0 g 3-Bromrifamycin-S, 1,0 g Natriumacetat, 1,0 g p-Hydroxybenzaldehyd und 30 ml Acetonitril wird im Druckrohr 2 Stunden auf 100° erhitzt und eingedampft. Der Rückstand wird in Äthylacetat aufgenommen, mit wäßriger Zitronensäurelösung gewaschen, getrocknet und eingedampft. Chromatographie an Kieselgel mit Methylenchlorid-Aceton (98 : 2) als Elutionsmittel liefert zuerst Spuren raschwandernden Materials und dann, als Hauptfraktion, 3-(4-Formylphenoxy)-rifamycin-S, das nach dem Eindampfen als gelber Schaum resultiert. 100-MHz-$^1$H-NMR-Spektrum ($CDCl_3$): Signale der aromat. H in AB-Spektrum zentriert bei 7,07 und 7,8 (J* ~ 9 Hz, 4 H); Aldehydsignal bei 12,7 (S, 1 H) ppm. IR-Spektrum ($CH_2Cl_2$): 3475 (OH), 3380 (NH), 2730 (OH), 1740 (5-Ring-CO), 1705 (Ester, CHO), 1675 (Amid I), 1645 (Chinon), 1620 (Chinon), 1600 (C = C Ringschwingung) $cm^{-1}$ usw; UV-Spektrum (0,0-N alkoholische Salzsäure, nm/$\varepsilon_{max}$) 214/38000, 269/34000, 345/6400, 400 (Schulter).

## Beispiel 8

Bei gleicher Arbeitsweise, wie in Beispiel 1 beschrieben, jedoch unter Verwendung von Natriumcarbonat statt des Phosphatpuffers, wird auf die beschriebene Weise 25-O-Desacetyl-3-hydroxyrifamycin-S als gelbes Pulver erhalten. Das 100-MHz-$^1$H-NMR-Spektrum ($CDCl_3$ ist analog dem des 3-Hydroxyrifamycin-S, jedoch fehlt das bei 2,1 ppm gelegene Signal der $C\underline{H}_3CO \cdot O$-Gruppe.

**Patentansprüche**

1. Rifamycin-S-Derivat der Formel (I)

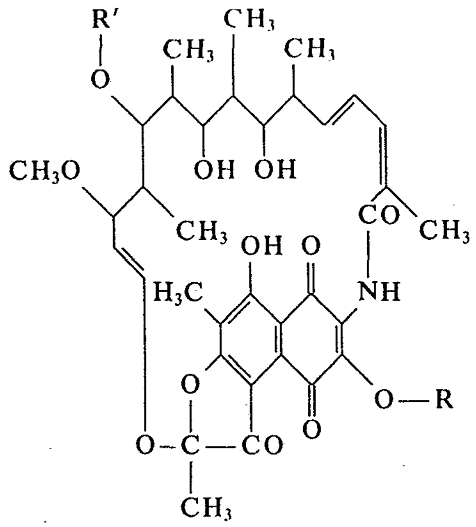

(I)

oder ein entsprechendes Derivat der SV-Reihe, worin R und R' je ein Wasserstoffatom oder R' Acetyl und R ein Rest Alk, Ar, Alk—CO—, Ar—CO— oder H—CO— ist, wobei Alk einen Alkylrest mit 1—7 C-Atomen, 2-Methoxyäthyl, 2-Äthoxyäthyl, 2-Hydroxyäthyl, 2- oder 3-Hydroxypropyl oder 4-Hydroxybutyl, in welchen die Hydroxylgruppe auch durch Ameisensäure oder eine $C_1$—$C_7$-Alkan-carbonsäure verestert werden kann, und Ar einen gegebenenfalls durch $C_1$—$C_7$-Alkyl, $C_1$—$C_7$-Alkoxy, Methylendioxy oder Formyl substituierten Phenylrest darstellt.

2. Eine Verbindung gemäß Anspruch 1, die aus der Gruppe bestehend aus 3-Phenoxyrifamycin-S, 3-Acetoxyrifamycin-S, 3-Benzoyloxyrifamycin-S, 3-(4-Formylphenoxy)-rifamycin-S und 25-O-Desacetyl-3-hydroxyrifamycin-S ausgewählt ist.

3. 3-Methoxyrifamycin-S.

4. 3-(2-Methoxyäthoxy)-rifamycin-S.

5. Eine Verbindung gemäß einem der Ansprüche 1 bis 4 zur Verwendung als Antibiotikum.

6. Ein pharmazeutisches Präparat enthaltend mindestens eine der Verbindungen gemäß einem der Ansprüche 1 bis 4 als Wirkstoff im Gemisch mit mindestens einem pharmazeutisch verträglichen Hilfsstoff.

7. Verfahren zur Herstellung eines im Anspruch 1 definierten Rifamycin-S-Derivats der Formel (I), eines entsprechenden Derivats der SV-Reihe, sowie des 3-Hydroxyrifamycin-S, dadurch gekennzeichnet, daß man 3-Bromrifamycin-S der Formel (II)

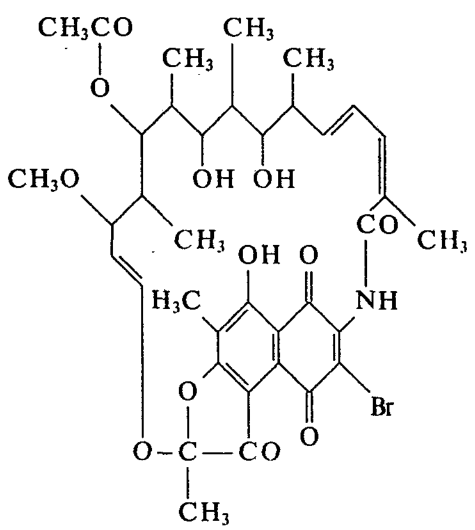

(II)

mit einer Verbindung der Formel ROH (III), worin R Wasserstoff bedeutet oder eine im Anspruch 1 angegebene Bedeutung hat, oder einem Salz davon, in neutralem bis basischem Milieu und Temperaturen zwischen 20° bis 140° und gegebenenfalls in Anwesenheit eines Bromwasserstoff-bindenden Mittels und/oder eines organischen Lösungsmittels behandelt und gewünschtenfalls ein erhaltenes Produkt

der S-Reihe zu einem entsprechenden Derivat der SV-Reihe reduziert und/oder ein erhaltenes Produkt der SV-Reihe zu einem entsprechenden Derivat der S-Reihe oxidiert.

8. Verfahren gemäß Anspruch 7, dadurch gekennzeichnet, daß man zur Herstellung einer Verbindung der Formel (I), worin R Wasserstoff bedeutet, den Ausgangsstoff der Formel (II) in einem wäßrigen Milieu in Gegenwart eines mit Wasser mischbaren polaren organischen Lösungsmittels bei pH 7,0 bis 10,0 erhitzt.

9. Verfahren gemäß Anspruch 8, dadurch gekennzeichnet, daß man durch Umsetzen im pH-Bereich um 9,0 25-O-Desacetyl-3-hydroxyrifamycin-S der Formel (I), worin je R und R' für Wasserstoff stehen, herstellt.

10. Verfahren gemäß Anspruch 8, dadurch gekennzeichnet, daß man durch Umsetzen im pH-Bereich zwischen 7,0 bis 8,0 3-Hydroxyrifamycin-S der Formel (I), worin R Wasserstoff und R' Acetyl bedeutet, herstellt.

11. Verfahren gemäß Anspruch 7, dadurch gekennzeichnet, daß man zur Herstellung einer Verbindung der Formel (I), worin R' Acetyl und R einen im Anspruch 1 definierten Rest Alk oder Ar bedeutet, den Ausgangsstoff der Formel (II) mit einem molaren Überschuß eines entsprechenden Alkohols der Formel AlkOH bzw. ArOH in Gegenwart von höchstens 2 Moläquivalenten eines Alkalimetallsalzes einer Carbonsäure, vorzugsweise Natriumacetat, umsetzt.

12. Verfahren gemäß Anspruch 7, dadurch gekennzeichnet, daß man zur Herstellung einer Verbindung der Formel (I), worin R' Acetyl und R einen Acylrest H—CO, Alk—CO— oder Ar—CO— bedeutet, in welchem Alk und Ar die im Anspruch 1 definierten Bedeutungen hat, den Ausgangsstoff der Formel (II) mit einem Alkalimetallsalz einer diesen Acylresten entsprechenden Carbonsäure in einem wasserfreien aprotischen polaren Lösungsmittel umsetzt.

13. Die nach dem Verfahren gemäß einem der Ansprüche 7 und 11 erhältlichen Verbindungen mit Ausnahme von 3-Hydroxyrifamycin-S.

## Claims

1. A rifamycin S derivative of the formula (I)

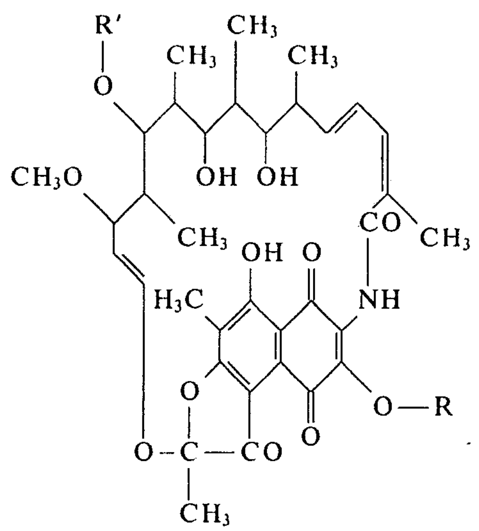

(I)

wherein each of R and R' is a hydrogen atom or R' is acetyl and R is a radical selected from Alk, Ar, Alk—CO—, Ar—CO— or H—CO—, in which Alk is a $C_1$—$C_7$alkyl radical, 2-methoxymethyl, 2-ethoxyethyl, 2-hydroxyethyl, 2- or 3-hydroxypropyl or 4-hydroxybutyl, in which the hydroxyl group can also be esterified by formic acid or a $C_1$—$C_7$alkanecarboxylic acid, and Ar is a phenyl radical which is unsubstituted or substituted by $C_1$—$C_7$alkyl, $C_1$—$C_7$alkoxy, methylenedioxy or formyl, or a corresponding derivative of the SV series.

2. A compound according to claim 1, which is selected from the group consisting of 3-phenoxyrifamycin S, 3-acetoxyrifamycin S, 3-benzoylrifamycin S, 3-(4-formylphenoxy)rifamycin S and 25-O-desacetyl-3-hydroxyrifamycin S.

3. 3-Methoxyrifamycin S.

4. 3-(2-Methoxyethoxy)rifamycin S.

5. A compound according to any one of claims 1 to 4 as an antibiotic.

6. A pharmaceutical composition containing at least one compound according to any one of claims 1 to 4 as active ingredient, in admixture with at least one pharmaceutically acceptable axcipient.

7. A process for the preparation of a rifamycin S derivative of the formula (I), as defined in claim 1, which comprises treating 3-bromorifamycin S of the formula (II)

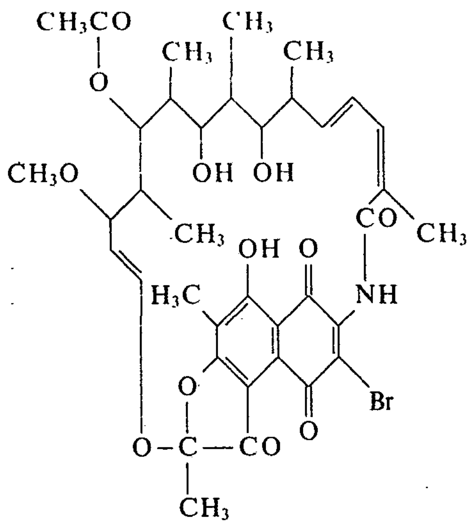

(II)

with a compound of the formula ROH (III), wherein R is hydrogen of has one the meanings assigned to it in claim 1, or a salt thereof, in a neutral to basic medium and in the temperature range from 20° to 140°C, and optionally in the presence of a hydrogen bromide acceptor and/or of an organic solvent, and, if desired, reducing a resultant compound of the S series to a corresponding derivative of the SV series, and/or oxidising a resultant compound of the SV series to a corresponding derivative of the S series.

8. A process according to claim 7 for the preparation of a compound of the formula (I), wherein R is hydrogen, which comprises heating the starting material of the formula (II), in aqueous medium and in the presence of a water-miscible polar organic solvent, in the pH range from 7.0 to 10.0.

9. A process according to claim 8 for the preparation of 25-O-desacetyl-3-hydroxyrifamycin S of the formula (I), wherein each of R and R' is hydrogen, which comprises carrying out the reaction in the pH range of about 9.0.

10. A process according to claim 8 for the preparation of 3-hydroxyrifamycin S of the formula (I), wherein R is hydrogen and R' is acetyl, which comprises carrying out the reaction in the pH range from 7.0 to 8.0.

11. A process according to claim 7 for the preparation of a compound of the formula (I), wherein R' is acetyl and R is a radical Alk or Ar as defined in claim 1, which comprises reacting the starting material of the formula (II) with a molar excess of a corresponding alcohol of the formula AlkOH or ArOH, in the presence of not more than 2 molar equivalents of an alkali metal salt of a carboxylic acid, preferably sodium acetate.

12. A process according to claim 7 for the preparation of a compound of the formula (I), wherein R' is acetyl and R is an acyl radical H—CO—, Alk—CO— or Ar—CO—, in which Alk and Ar are as defined in claim 1, which comprises reacting the starting material of the formula (II) with an alkali metal salt of a carboxylic acid corresponding to said acyl radicals, in an anhydrous aprotic polar solvent.

13. The compounds obtainable according to either of claims 7 or 11, with the exception of 3-hydroxy-rifamycin S.

**Revendications**

1. Dérivé en configuration S de la rifamycine, répondant à la formule (I)

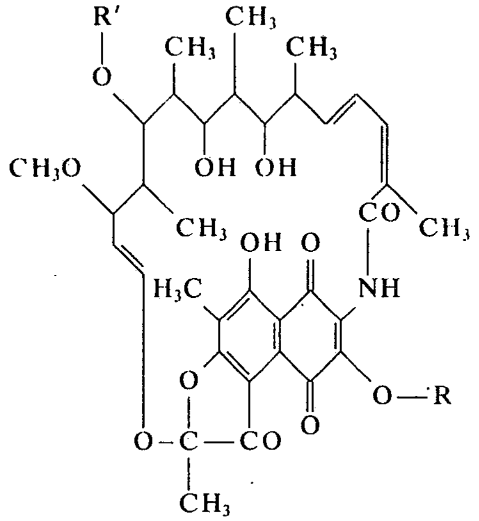

(I)

ou un dérivé correspondant de la série SV, dans lequel R et R' représentent chacun un atome d'hydrogène ou bien R' représente un groupe acétyle et R un reste Alk, Ar, Alk—CO—, Ar—CO— ou H—CO—, Alk étant un groupe alkyle en $C_1$—$C_7$, 2-méthoxyéthyle, 2-éthoxyéthyle, 2-hydroxyéthyle, 2- ou 3-hydroxypropyle ou 4-hydroxybutyle, dans lesquels le groupe hydroxy peut également être estérifié par l'acide formique ou par un acide (alcane en $C_1$—$C_7$)-carboxylique, et Ar représente un reste phényle éventuellement substitué par un groupe alkyle en $C_1$—$C_7$, alcoxy en $C_1$—$C_7$, méthylènedioxy ou formyle.

2. Un composé selon la revendication 1, choisi dans le groupe consistant en la 3-phénoxyrifamycine-S, la 3-acétoxyrifamycine-S, la 3-benzoyloxyrifamycine-S, la 3-(4-formylphénoxy)-rifamycine-S et la 25-O-désacétyl-3-hydroxyrifamycine-S.

3. La 3-méthoxyrifamycine-S.

4. La 3-(2-méthoxyéthoxy)-rifamycine-S.

5. Un composé selon l'une des revendications 1 à 4, pour l'utilisation en tant qu'antibiotique.

6. Une compositions pharmaceutique contenant au mois un des composés selon l'une des revendications 1 à 4, en tant que substance active en mélange avec au moins un produit auxiliaire acceptable pour l'usage pharmaceutique.

7. Procédé de préparation d'un dérivé en configuration S de la rifamycine, répondant à la formule (I), d'un dérivé correspondant de la série SV et de la 3-hydroxyrifamycine-S, caractérisé en ce que l'on traite la 3-bromorifamycine-S de formule (II):

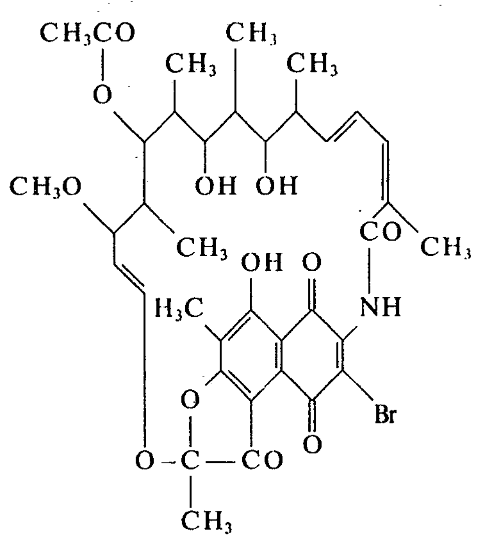

(II)

par un composé de formule ROH (III), dans laquelle R représente l'hydrogène ou a l'une des significations indiquées dans la revendication 1, ou un sel d'un tel composé, en milieu neutre à basique et à des températures de 20 à 140°, et le cas échéant en présence d'un agent fixant le bromure d'hydrogène et/ou d'un solvant organique, et si on le désire, on réduit un produit obtenu de la série S en un dérivé correspondant de la série SV et/ou on oxyde un produit obtenu de la série SV en un dérivé correspondant de la série S.

8. Procédé selon la revendication 7, caractérisé en ce que, pour préparer un composé de formule (I) dans laquelle R représente l'hydrogène, on chauffe le produit de départ de formule (II) dans un milieu aqueux en présence, d'un solvant organique polaire miscible à l'eau, à pH 7,0 à 10,0.

9. Procédé selon la revendication 8, caractérisé en ce que l'on prépare la 25-O-désacétyl-3-hydroxyrifamycine-S de formule (I) dans laquelle R et R' représentent chacun l'hydrogène par réaction à un pH d'environ 9,0.

10. Procédé selon la revendication 8, caractérisé en ce que l'on prépare la 3-hydroxyrifamycine-S de formule (I) dans laquelle R représente l'hydrogène et R' le groupe acétyle par réaction à un pH de 7,0 à 8,0.

11. Procédé selon la revendication 7, caractérisé en ce que, pour préparer un composé de formule (I) dans laquelle R' représente un groupe acétyle et R un reste Alk ou Ar défini dans la revendication 1, on fait réagir le produit de départ de formule (II) avec un excès molaire de l'alcool correspondant de formule AlkOH ou ArOH respectivement en présence d'un maximum de 2 équivalents molaires d'un sel de métal alcalin d'un acide carboxylique, de préférence l'acétate de sodium.

12. Procédé selon la revendication 7, caractérisé en ce que, pour préparer un composé de formule (I), dans laquelle R' représente un groupe acétyle et R un reste acyle H—CO, Alk—CO— ou Ar—CO— dans lequel Alk et Ar ont les significations indiquées dans la revendication 1, on fait réagir le produit de départ de formule (II) avec un sel de métal alcalin d'un acide carboxylique correspondant à ces restes acyle dans un solvant polaire aprotonique anhydre.

13. Les composés qu'on peut obtenir par le procédé selon l'une des revendications 7 et 11 à l'exception de la 3-hydroxyrifamycine-S.